# DEMANDE DE BREVET EUROPEEN

(11) **EP 2 065 026 A1**
(43) Date de publication de la demande: **03.06.2009**
(21) Numéro de dépôt: 09151186.5
(22) Date de dépôt: 13.10.2005
(51) Int. Cl.: A61K 8/40, A61K 8/36, A61K 8/368, A61Q 5/06

(54) **Composition comprenant, dans un milieu cosmétiquement acceptable anhydre, des monomères électrophiles et un acide, et son utilisation pour le traitement cosmétique des cheveux**

(30) Priorité: 13.10.2004 FR 0410813
(62) Demande divisionnaire de: 05292142.6
(71) Demandeur: L'Oreal, 75008 Paris (FR)
(72) Inventeur: VIC, Gabin, 60280, Venette (FR); GOURLAOUEN, Luc, 92600, Asnières (FR); LIVOREIL, Aude, 75006, Paris (FR); BRUN, Gaëlle, 75015, Paris (FR); GIROUD, Franck, 92110, Clichy (FR)
(74) Mandataire: Dossmann, Gérard

(57) **Abrégé**

La présente invention concerne une composition comprenant, dans un milieu cosmétiquement acceptable anhydre, au moins un monomère électrophile et au moins un acide organique non-réducteur comportant de 1 à 12 atomes de carbone, et son utilisation pour le traitement cosmétique des cheveux.

Elle concerne aussi un procédé de traitement cosmétique des cheveux mettant en oeuvre ladite composition.

## Description

La présente invention est relative à une composition cosmétique comprenant au moins un monomère électrophiles et au moins un acide particulier, dans un milieu cosmétiquement acceptable anhydre.

La présente invention concerne également l'utilisation d'une telle composition pour le traitement cosmétique des cheveux, et un procédé de traitement cosmétique mettant en oeuvre une telle composition.

Dans le domaine de la cosmétique, on cherche à modifier les propriétés superficielles des cheveux, notamment pour leur apporter un effet conditionnant comme de la douceur, ou de la brillance. Pour ce faire, on utilise généralement des compositions cosmétiques à base d'agents de conditionnement tels que des silicones ou des polymères ayant une forte affinité pour les cheveux.

Cependant, ces agents de conditionnement ont tendance à s'éliminer au cours de lavage avec des shampoings, rendant nécessaire le renouvellement des applications des compositions sur les cheveux.

Pour accroître la rémanence de dépôt de polymères, il peut être envisagé d'effectuer une polymérisation radicalaire de certains monomères directement sur les cheveux. Toutefois, on constate une forte dégradation des fibres capillaires liées probablement aux amorceurs de polymérisation et les cheveux ainsi traités sont difficilement démêlables.

La Demanderesse a trouvé de manière surprenante qu'en associant un milieu cosmétiquement acceptable anhydre particulier et au moins un acide particulier à au moins un monomère électrophile tel que décrit ci-dessous, il était possible d'obtenir un conditionnement et une brillance améliorés des cheveux, et ce de manière durable.

En effet, une composition comprenant une telle association permet de maintenir la douceur et la brillance apportées à la chevelure par ladite composition, et ceci sans ré-application, même après plusieurs lavages de la chevelure.

L'application d'une composition comprenant une telle association conduit à la formation in situ d'un revêtement lubrifiant et brillant, rémanent notamment aux shampoings.

En outre, les cheveux restent de manière surprenante parfaitement individualisés et peuvent être coiffés sans problème.

L'invention a donc pour objet une composition cosmétique comprenant au moins un monomère électrophile et au moins un acide organique non-réducteur comportant de 1 à 12 atomes de carbone, dans un milieu cosmétiquement acceptable anhydre choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀-C₃₀ et leurs mélanges.

Un autre objet de la présente invention consiste en l'utilisation de ladite composition pour le traitement cosmétique des cheveux.

L'invention a encore pour objet un procédé de traitement cosmétique des cheveux, mettant en oeuvre ladite composition.

D'autres objets, caractéristiques, aspects et avantages de l'invention apparaîtront encore plus clairement à la lecture de la description et des exemples qui suivent.

Selon l'invention, la composition comprend dans un milieu cosmétiquement acceptable anhydre, au moins un monomère électrophile et au moins un acide organique non-réducteur comportant de 1 à 12 atomes de carbone.

Par "non-réducteur", on entend au sens de la présente invention un composé dont le potentiel d'oxydo-réduction standard à 25°C (par rapport à l'électrode à hydrogène) est supérieur à 0 volt.

Par milieu cosmétiquement acceptable, on entend un milieu compatible avec les cheveux.

On entend par « milieu anhydre », un milieu contenant moins de 1 % en poids d'eau par rapport au poids total de la composition.

Le milieu cosmétiquement acceptable est choisi parmi les huiles organiques ; les silicones telles que par exemple les silicones volatiles, les gommes ou huiles de silicones aminés ou non et leurs mélanges ; les huiles minérales ; les huiles végétales telles que par exemple les huiles d'olive, de ricin, de colza, de coprah, de germe de blé, d'amande douce, d'avocat, de macadamia, d'abricot, de carthame, de noix de bancoulier, de camélina, de tamanu, de citron ; les cires ; ou encore des composés organiques tels que des alcanes en C₅-C₁₀, l'acétone, la méthyléthylcétone, les esters d'acides en C₁-C₂₀ et d'alcools en C₁-C₈ tels que par exemple l'acétate de méthyle, l'acétate de butyle, l'acétate d'éthyle et le myristate d'isopropyle, le diméthoxyéthane, le diéthoxyéthane, les alcools gras en C₁₀-C₃₀ tels que par exemple l'alcool laurique, l'alcool cétylique, l'alcool stéarylique et l'alcool béhénylique, les acides gras en C₁₀-C₃₀ tels que par exemple l'acide laurique et l'acide stéarique, les amides gras en C₁₀-C₃₀ tels que part exemple le diéthanolamide laurique, les esters d'alcools gras en C₁₀-C₃₀ tels que par exemple les benzoates d'alcool gras en C₁₀-C₃₀ et leurs mélanges.

De préférence, les composés organiques constituant le milieu sont choisis parmi les composés liquides à la température de 25°C et sous 10⁵ Pa (760mm de Hg).

Le milieu cosmétiquement acceptable utilisé de préférence est choisi parmi les huiles d'olive, de ricin, de colza, de coprah, de germes de blé, d'amande douce, d'avocat, de macadania, d'abricot, de carthame, de noix de bancoulier, de camélina, de tamanu, de citron ; l'huile de polybutène, l'isononanoate d'isononyle, le malte d'isostéaryle, le tétra-isostéarate de pentaérythrityle, le trimélate de tridécyle, le mélange cyclopentasiloxane (14,7% en poids)/polydiméthylsiloxane dihydroxylé en positions α et ω (85,3% en poids), ou leurs mélanges.

Les acides organiques utilisables dans l'invention peuvent être des acides carboxyliques, des acides sulfoniques, des acides phosphoniques, des acides phosphiniques, des mono- ou di-esters phosphoriques. Ils comprennent tous de 1 à 12 atomes de carbone.

Les acides utilisables dans la présente invention présentent de préférence un pKa compris entre 0 et 6.

A titre d'exemples d'acide organique selon l'invention, on peut notamment citer l'acide acétique, l'acide formique, l'acide propionique, l'acide butyrique, l'acide benzoïque, les acides mono-, di- ou trichloroacétiques, l'acide salicylique, l'acide trifluoroacétique, les acides benzène- et toluène-sulfoniques, les acides méthylphosphonique et méthylphosphinique, l'acide diméthylphosphinique et le monobutylester de l'acide phosphonique.

De préférence l'acide ne comporte pas de sous-unité PO₄.

L'acide particulièrement préféré dans la présente invention est l'acide acétique.

L'acide ou le mélange d'acides tels que décrits ci-dessus, est présent dans les compositions cosmétiques de l'invention, en une concentration comprise entre 0,001 et 20% en poids, de préférence entre 0,002 et 10% en poids, et mieux encore entre 0,005 et 5 % en poids par rapport au poids total de la composition.

Par monomère électrophile, on entend un monomère capable de polymériser par polymérisation anionique en présence d'un agent nucléophile tel que par exemple, les ions hydroxyles (OH⁻) contenus dans l'eau.

Par polymérisation anionique, on entend le mécanisme défini dans l'ouvrage "Advanced Organic Chemistry", Third Edition de Jerry March, pages 151 à 161.

Le ou les monomères électrophiles présents dans la composition de l'invention peuvent être choisis parmi :
(i) les dérivés benzylidene malononitrile (A), le 2-(4-chloro-benzylidene)-malononitrile (A1), le 2-cyano-3-phényl acrylate d'éthyle (B), le 2-cyano-3-(4-ehloro-phényi) acrylate d'éthyle (B1) tels que décrits dans Sayyah, J. Polymer Research, 2000, p97 : 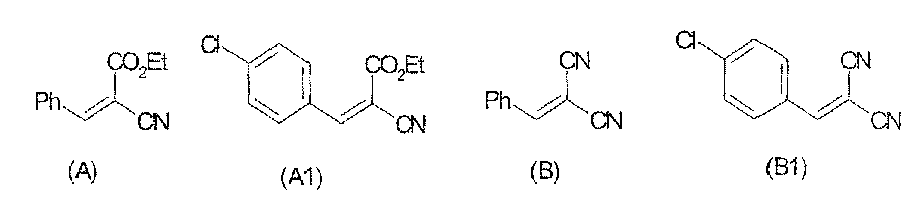
(ii) les dérivés de méthylidenemalonates comme :
   - le 2-méthylene-malonate de diéthyle (C) tel que décrit par Hopff, Makromoleculare Chemie, 1961, p95, par De Keyser, J. Pharm. Sci, 1991, p67 et par Klemarczyk, Polymer, 1998, p173 : 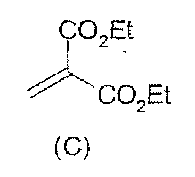
   - le 2-éthoxycarbonylméthyleneoxycarbonyl acrylate d'éthyle (D) tel que décrit par Breton, Biomaterials, 1998, p271 et Couvreur, Pharmaceutical Research, 1994, p1270 : 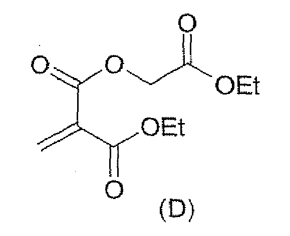
(iii) les dérivés itaconate et itaconimide comme :
   - l'itaconate de diméthyle (E) tel que décrit par Bachrach, European Polymer Journal, 1976, p563 : 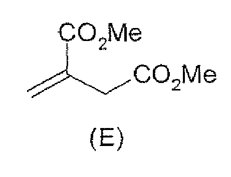
   - le N-butyl itaconimide (F), le N-(4-tolyl) itaconimide (G), le N-(2-ethylphenyl) itaconimide (H), le N-(2,6-diethylphenyl) itaconimide (I) tel que décrits par Wanatabe, J.Polymer Science : Part A :Polymer chemistry, 1994, p2073 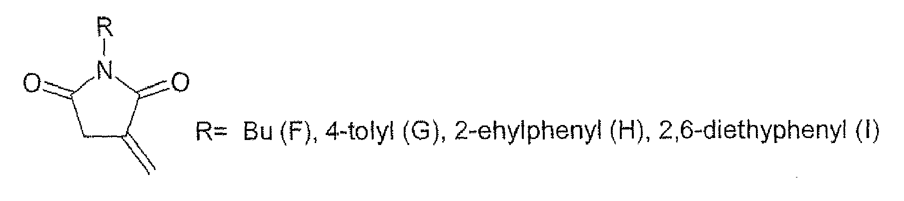
(iv) les dérivés α-(methylsulfonyl)acrylates de méthyle (K), α-(methylsulfonyl)acrylates d'éthyle (L), α-(tert-butylsulfonyl)acrylates de méthyle (M), α-(methylsulfonyl)acrylates de tert-butyle (N), α-( tert-butylsulfonyl)acrylates de tert-butyle (O) tel que décrits par Gipstein, J.Org.Chem, 1980, p1486 et les dérivés 1,1-bis-(methylsulfonyl)ethylene (P), 1-acetyl-1-methyl sulfonyl ethylene (Q) , α-(methylsulfonyl) vinyl sulfonate de methyle (R) , α-methylsulfonylacrylonitrile (S) tel que décrits par Shearer, US patent US2748050 : 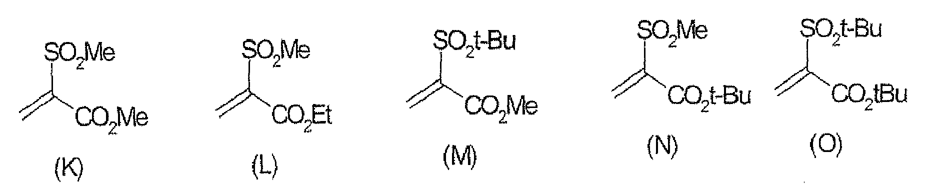 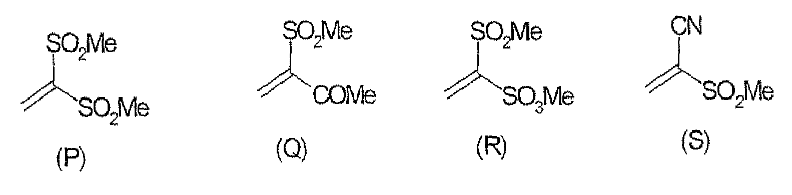
(v) les dérivés méthyl vinyl sulfone (T) et phényl vinyl sulfone (U) tel que décrits par Boor , J.Polymer Science, 1971, p249 : 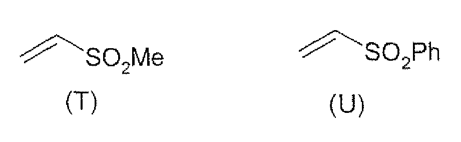
(vi) le dérivé phénol vinyl sulfoxide (V) tel que décrit par Kanga, Polymer preprints (ACS, Divison of Polymer Chemistry), 1987, p322 : 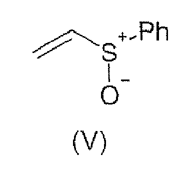
(vii) le dérivé 3-methyl-N-(phenylsulfonyl)-1-aza-1,3-butadiene (W) tel que décrit par Bonner, Polymer Bulletin, 1992, p517 : 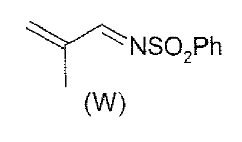
(viii) les dérivés acrylates et acrylamides comme :
   - le N-propyl-N-(3-triisopropoxysilylpropyl)acrylamide (X) et N-propyl-N-(3-triethoxysilylpropyl)acrylamide (Y) tel que décrit par Kobayashi, Journal of Polymer Science, Part A: Polymer Chemistry, 2005, p2754 : 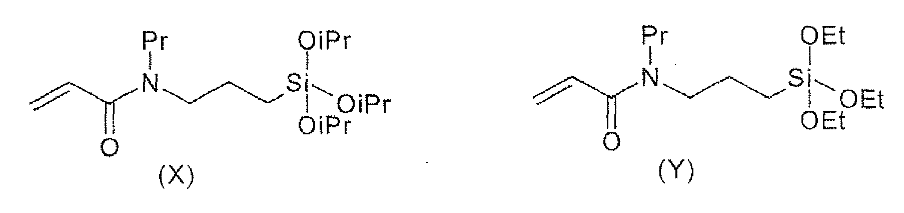
   - le 2-hydroxyethyl acrylate (Z) et le 2-hydroxyethyl méthacrylate (AA) tel que décrits par Rozenberg, International Journal of Plastics Technology, 2003, p17 : 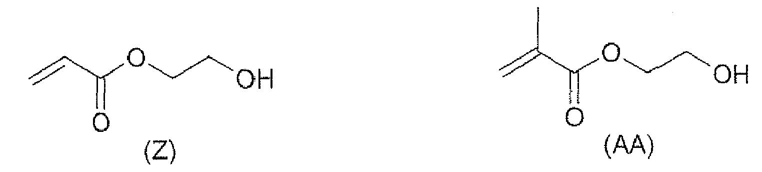
   - le N-butyl acrylate (AB) tel que décrit par Schmitt, Macromolecules, 2001, p2115, et le tert-butyl acrylate (AC) tel que décrit par Ishizone, Macromolecules, 1999, p955 :

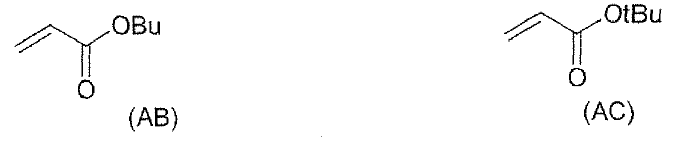

Le monomère électrophile, ou électro-attracteur, utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier, ces monomères présentent au moins deux groupes électro-attracteurs.

A titre d'exemple de monomères électrophiles présentant au moins deux groupes électro-attracteurs, on peut citer les monomères de formule (I) : 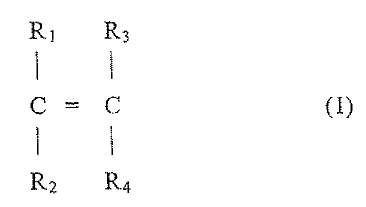 dans laquelle:
R₁ et R₂ désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
   - un résidu polyorganosiloxane modifié ou non,
   - un groupement polyoxyalkylène,
      R₃ et R₄ désignent chacun, indépendamment l'un de l'autre, un groupe électro-attracteur (ou inductif-attacteur) choisi de préférence parmi les groupements -N(R)₃⁺, -S(R)₂⁺, -SH₂⁺, -NH₃⁺, -NO₂, -SO₂R, -C≡N,
      -COOH, -COOR, -COSR, -CONH₂, -CONHR, -F, -Cl, -Br, -I, -OR,
      -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle tels que phényle, ou les groupements aryloxy tels que phénoxyloxy,
      R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène et un résidu de polymère pouvant être obtenu par polymérisation radicalaire ou par polycondensation ou par ouverture de cycle, R' désignant un groupe alkyle en C₁-C₁₀.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, mieux encore de 1 à 10 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)ₙ-(CF₂)ₘ-CF₃ ou -(CH₂)ₙ-(CF₂)ₘ-CHF₂ avec n=1 à 20 et m= 1 à 20.

Les substituants R₁ à R₄ peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthanes.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et les groupements de type esters gras.

Parmi les monomères précédemment cités, sont préférés les monomères de la famille des cyanoacrylates et leurs dérivés de formule (II) : 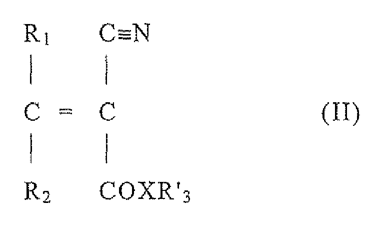
X désignant NH,S,O,
R₁ et R₂ ayant les mêmes significations que précédemment,
R'₃ pouvant désigner un atome d'hydrogène ou un groupe R tel que défini pour la formule (I).

De préférence, X désigne O.

A titre de composés de formule (II), on peut citer les monomères :
a) appartenant à la famille des 2-cyanoacrylates de polyfluoroalkyle en C₁₋₂₀ tels que :
   l'ester 2,2,3,3-tétrafluoropropylique de l'acide 2-cyano-2-propénoïque de formule : 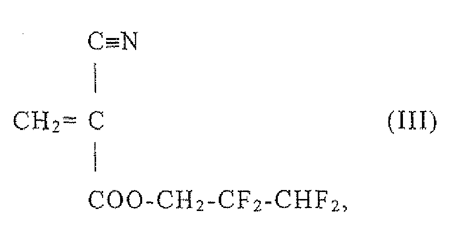 ou encore l'ester 2,2,2-trifluoroéthylique de l'acide 2-cyano-2-propénoïque de formule : 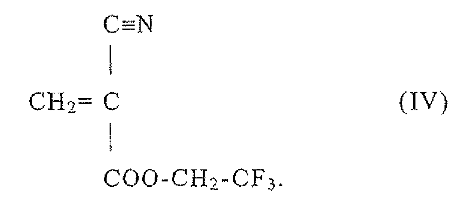
b) les cyanoacrylates d'alkyle en C₁-C₁₀ ou d'(alcoxy en C₁-C₄)(alkyle en C₁-C₁₀).

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b).

Les monomères les plus particulièrement préférés sont ceux de formule V et leurs mélanges : 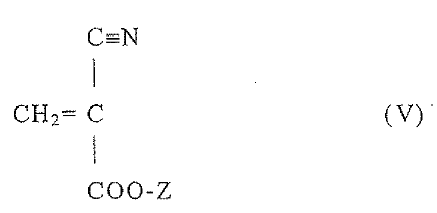 dans laquelle : Z=-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C, de préférence entre 10 et 80°C, de préférence 20 à 80°C, ce qui n'empêche pas de terminer l'application par un séchage au casque, un brushing ou passage au fer plat ou à friser.

Les compositions mises en oeuvre conformément à l'invention ont généralement une concentration en monomère électrophile selon l'invention comprise entre 0,001 et 80 % en poids, de préférence entre 0,002 et 75 % en poids, plus particulièrement entre 0,1 et 40 % et encore plus préférentiellement entre 1 et 20 % en poids par rapport au poids total de la composition.

On peut également introduire dans les compositions des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que le monoéthyléther d'hydroquinone, la tert-butyihydroquirzone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfone, les alkyl sulfoxydes, les alkyl sulfures, les mercaptans, le 3-sulfolène et leurs mélanges. Les groupements alkyle désignent de préférence des groupements ayant 1 à 6 atomes de carbone.

La quantité d'inhibiteur(s) peut aller de 10 ppm à 20%, et plus préférentiellement de 10 ppm à 5% et encore plus préférentiellement de 10 ppm à. 1 % en poids par rapport au poids total de la composition.

Les compositions conformes à l'invention peuvent également contenir au moins un agent utilisé habituellement en cosmétique, choisi, par exemple, parmi des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des polymères fixants ou non, des polyols, des protéines, des vitamines, des colorants directs ou d'oxydation, des agents nacrants, des propulseurs, et des épaississants minéraux ou organiques tels que le benzylidène-sorbitol et les N-acylaminoacides.

Ces agents peuvent être éventuellement encapsulés. La capsule peut être de type polycyanoacrylate.

La composition selon l'invention est utilisée sur les cheveux, de préférence en présence d'un agent nucléophile, pour leur traitement cosmétique.

Un procédé de traitement cosmétique selon l'invention comprend l'application d'une composition telle que définie ci-dessus, sur les cheveux, en présence d'un agent nucléophile tel que défini ci-dessous.

Les agents nucléophiles susceptibles d'initier la polymérisation anionique sont des systèmes connus en eux-mêmes, capables de générer un carbanion au contact d'un monomère électrophile. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

Les agents nucléophiles particulièrement préférés sont les ions hydroxyle, notamment ceux présents dans l'eau. Cette eau peut être apportée par une humidification préalable des cheveux.

Selon un mode préféré de réalisation, la composition comprenant le monomère électrophile est exempte d'agent nucléophile.

Selon un autre mode préféré de réalisation, l'agent nucléophile est apporté par une seconde composition, appliquée sur ou sous le dépôt formé par l'application de la composition comprenant le monomère électrophile.

Il est également possible, afin de moduler la cinétique de réaction, de préalablement humidifier les cheveux à l'aide d'une solution aqueuse dont le pH a été ajusté à l'aide d'une base, d'un acide ou d'un mélange acide/base. L'acide et/ou la base peuvent être inorganiques ou organiques.

Il est également possible de moduler la cinétique de polymérisation par voie anionique en pré-imprégnant les cheveux, à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie des cheveux, par transformation chimique de la matière kératinique.

A titre d'exemple de transformation chimique, on peut citer la réduction des ponts di-sulfure composant en partie la kératine en thiols, avant application de la composition de l'invention. De façon non exhaustive, on peut citer comme réducteurs des ponts di-sulfure composant en partie la kératine, les composés suivants:
- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylate tels qu'ils sont décrits dans le brevet US 6,224,622.

Afin d'améliorer entre autres l'adhésion du poly(cyanoacrylate) formé in situ, on peut pré-traiter les cheveux avec tous types de polymères, ou réaliser un traitement capillaire avant application de la composition de l'invention, comme une coloration directe ou d'oxydation, une permanente ou encore un défrisage.

L'application des compositions telles que décrites ci-dessus peut être suivie ou non d'un rinçage.

Ces compositions peuvent se présenter sous la forme de lotion, de spray, de mousse, et être appliquées comme shampoing ou après-shampoing.

Un autre objet de l'invention consiste en un kit comprenant une première composition contenant au moins un monomère électrophile tel que défini ci-dessus et éventuellement au moins un inhibiteur de polymérisation anionique et/ou radicalaire tel que défini ci-dessus, ainsi qu'une deuxième composition comprenant, dans un milieu cosmétiquement acceptable anhydre choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀-C₃₀ et leurs mélanges, au moins un acide organique non-réducteur comportant de 1 à 12 atomes de carbone tel que défini ci-dessus.

L'invention a encore pour objet un procédé de traitement cosmétique des cheveux, comprenant au moins deux étapes, une étape comprenant l'application de l'acide organique non-réducteur comportant de 1 à 12 atomes de carbone tel que défini ci-dessus, et une autre étape comprenant l'application d'au moins un monomère électrophile tel que défini ci-dessus, l'ordre des étapes étant indifférent.

Un mode de réalisation particulier de l'invention consiste en ce que l'application de l'acide organique non-réducteur se fait avant l'application d'au moins un monomère électrophile.

Les exemples suivants sont donnés à titre illustratif de la présente invention.

Dans les exemples suivants, toutes les quantités sont indiquées en pour cent en poids de matière active par rapport au poids total de la composition, sauf indication contraire.

### EXEMPLES

### Exemple 1 :

On a préparé une crème de brillance selon l'invention (exemple 1) et une crème de brillance comparative (exemple comparatif 1) à partir des ingrédients suivants :

| | Ex. 1 | Ex. Comp. 1 |
|---|---|---|
| 2-cyanoacrylate de N-octyle | 5% | 5% |
| Cyclopentasiloxane | 47,35% | 47,4% |
| Cyclopentasiloxane dimethicone copolyol | 47,4% | 47,4% |
| Acide acétique | 0,05 % | - |
| parfum | 0,2 % | 0,2 % |

### Mode d'application

Des mèches constituées de 2,7g de cheveux moyennement sensibilisés sont humidifiées à l'aide de 1ml d'eau par mèche. 2g de chacune des compositions des exemple 1 et exemple comparatif 1, sont appliqués sur ces mèches humidifiées. Après application, les mèches de cheveux sont séchées au casque 30 minutes à 40°C.

Pour chaque mèche, le toucher et la brillance des cheveux sont évalués par un panel de 10 personnes. Une mèche vierge de même nature est utilisée comme référence.

L'évaluation tactile et visuelle des diverses mèches de cheveux est répétée avec la même procédure après avoir réalisé successivement 5 shampooings commercialisés sous la dénomination DOP camomille.

Les résultats d'évaluation sensorielle sont indiqués dans le tableau suivant :

| Nature du traitement | Ex. 1 | Ex. comp. 1 |
|---|---|---|
| Après | Douceur 5 | Douceur 3 |
| application | Brillance 5 | Brillance 3 |
| Après 5 | Douceur 4 | Douceur 2 |
| shampoings | Brillance 4 | Brillance 2 |

La notation est :
0 = équivalent à la mèche non traitée,
5 = très supérieur à la mèche non traitée.

La douceur et la brillance apportées par la composition selon l'invention sont supérieures à celles apportées par la composition de l'exemple comparatif 1 juste après l'application de la composition.

Après 5 shampooings, l'apport de douceur et de brillance est mieux conservé lorsque les mèches sont traitées avec la composition selon l'invention, de l'exemple 1.

### Exemples 2 à 7 :

Les compositions détaillées ci-après illustrent d'autres exemples de crèmes de brillance conformes à l'invention.

### Exemple 2 :

| | Pourcentage massique |
|---|---|
| Methylheptylcyanoacrylate (1) | 10% |
| Cyclopentasiloxane | 44.55% |
| Cyclopentasiloxane dimethicone copolyol | 45% |
| Acide acétique | 0,25 % |
| Parfum | 0,2 % |

| | |
|---|---|
| (1) commercialisé par la société Chemence | |

### Exemple 3 :

| | Pourcentage massique |
|---|---|
| Ethoxyethylcyanoacrylate (1) | 10% |
| Cyclopentasiloxane | 43.8% |
| Cyclopentasiloxane dimethicone copolyol | 45% |
| Acide acétique | 1 % |
| Parfum | 0,2 % |

| | |
|---|---|
| (1) EO 460 commercialisé par la société Tong Shen | |

### Exemple 4 :

| | Pourcentage massique |
|---|---|
| Butylcyanoacrylate (1) | 10% |
| Cyclopentasiloxane | 43.8% |
| Cyclopentasiloxane dimethicone copolyol | 45% |
| Acide acétique | 1 % |
| Parfum | 0,2 % |

| | |
|---|---|
| (1) B 60 commercialisé par la société Tong Shen | |

### Exemple 5 :

| | Pourcentage massique |
|---|---|
| Ethylhexylcyanoacrylate (1) | 10% |
| Cyclopentasiloxane | 44.55% |
| Cyclopentasiloxane dimethicone copolyol | 45% |
| Acide acétique | 0.25 % |
| Parfum | 0,2 % |

| | |
|---|---|
| (1) O 60 commercialisé par la société Tong Shen | |

### Exemple 6 :

| | Pourcentage massique |
|---|---|
| Methylheptylcyanoacrylate (1) | 9 % |
| Ethylhexylcyanoacrylate (2) | 1 % |
| Cyclopentasiloxane | 44.55% |
| Cyclopentasiloxane dimethicone copolyol | 45% |
| Acide acétique | 0,25 % |
| Parfum | 0,2 % |

| | |
|---|---|
| (1) commercialisé par la société Chemence (2) 0-60 commercialisé par la société Tong Shen | |

### Exemple 7 :

| | Pourcentage massique |
|---|---|
| Methylheptylcyanoacrylate (1) | 7 % |
| Butylcyanoacrylate (2) | 3 % |
| Cyclopentasiloxane | 44.55% |
| Cyclopentasiloxane dimethicone copolyol | 45% |
| Acide acétique | 0,25 % |
| Parfum | 0,2 % |

| | |
|---|---|
| (1) commercialisé par la société Chemence (2) B-60 commercialisé par la société Tong Shen | |

## Revendications

1. Composition pour le traitement cosmétique des cheveux comprenant au moins un monomère électrophile et au moins un acide organique non-réducteur comportant de 1 à 12 atomes de carbone, dans un milieu cosmétiquement acceptable anhydre choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀-C₃₀ et leurs mélanges.

2. Composition selon la revendication 1, **caractérisée en ce que** l'acide organique est choisi parmi les acides carboxyliques, les acides sulfoniques, les acides phosphoniques, les acides phosphiniques et les mono- ou di-esters phosphoriques.

3. Composition selon la revendication 1 ou 2, **caractérisée en ce que** l'acide est choisi parmi l'acide acétique, l'acide formique, l'acide propionique, l'acide butyrique, l'acide benzoïque, les acides mono-, di- ou trichloroacétiques, l'acide salicylique, l'acide trifluoroacétique, les acide benzène- ou toluène-sulfoniques, les acides méthylphosphonique et méthylphosphinique, l'acide diméthylphosphinique et le monobutylester de l'acide phosphorique.

4. Composition selon la revendication 3, **caractérisée en ce que** l'acide est l'acide acétique.

5. Composition selon la revendication 1, **caractérisée par le fait que** le ou les monomères sont choisis parmi les 2-cyanoacrylates de polyfluoroalkyle en C₁₋₂₀, les cyanoacrylates d'alkyle en (C₁-C₁₀) ou d'(alcoxy en C₁-C₄)(alkyle en C₁-C₁₀).

6. Composition selon la revendication 5, **caractérisée en ce que** le ou les monomères sont choisis parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

7. Composition selon la revendication 6, **caractérisée en ce que** le ou les monomères électrophiles répondent à la formule (V) : 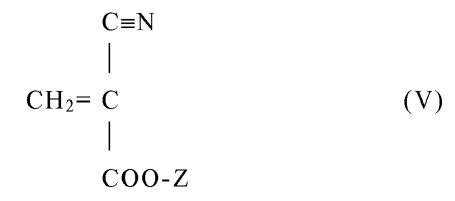 dans laquelle : Z=-(CH₂)₇-CH₃,
- CH(CH₃)-(CH₂)₅-CH₃,
- CH₂-CH(C₂H₅)-(CH₂)₃-CH₃,
- (CH₂)₅-CH(CH₃)-CH₃,
- (CH₂)₄-CH(C₂H₅)-CH₃.

8. Composition selon l'une quelconque des revendications précédentes, **caractérisée en ce que** la composition contient des inhibiteurs de polymérisation.

9. Utilisation de la composition selon l'une quelconque des revendications précédentes, pour le traitement cosmétique des cheveux.

10. Utilisation selon la revendication 9, en présence d'un agent nucléophile, choisi parmi les composés moléculaires, les oligomères, les dendrimères ou polymères possédant des fonctions nucléophiles choisies parmi : R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

11. Utilisation selon la revendication 10, **caractérisée en ce que** l'agent nucléophile est constitué d'ions hydroxyle, notamment ceux présents dans l'eau.

12. Procédé de traitement cosmétique des cheveux, comprenant l'application sur les cheveux d'une composition selon l'une quelconque des revendications 1 à 8, en présence d'un agent nucléophile.

13. Procédé selon la revendication 12, **caractérisé en ce que** l'on applique la composition sur les cheveux préalablement humidifiés à l'aide d'une solution aqueuse dont le pH a été ajusté à l'aide d'une base, d'un acide ou d'un mélange acide/base.

14. Kit comprenant une première composition contenant au moins un monomère électrophile tel que défini dans l'une quelconque des revendications 5 à 7, et éventuellement au moins un inhibiteur de polymérisation anionique et/ou radicalaire, ainsi qu'une deuxième composition comprenant, dans un milieu cosmétiquement acceptable anhydre choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀-C₃₀ et leurs mélanges, au moins un acide organique non-réducteur comportant de 1 à 12 atomes de carbone tel que défini dans l'une quelconque des revendications 2 à 4.

15. Procédé de traitement cosmétique des cheveux comprenant au moins deux étapes, une étape comprenant l'application d'au moins un acide organique non-réducteur comportant de 1 à 12 atomes de carbone tel que défini dans l'une quelconque des revendications 2 à 4, et une autre étape comprenant l'application d'au moins un monomère électrophile tel que défini dans l'une quelconque des revendications 5 à 7.
